# EUROPEAN PATENT SPECIFICATION

(11) **EP 3 527 082 B1**
(45) Date of publication and mention of the grant of the patent: **15.11.2023**
(21) Application number: 17860080.5
(22) Date of filing: 22.09.2017
(51) Int. Cl.: A23L 29/00, A61K 31/70, A61K 38/47, A23L 33/16, A23L 33/175

(54) **DIETARY COMPLEMENT COMPOSITION**
NAHRUNGSERGÄNZUNGSAMMENSETZUNG
COMPOSITION DE COMPLÉMENT DIÉTÉTIQUE

(30) Priority: 13.10.2016 ES 201631322
(43) Date of publication of application: 21.08.2019
(73) Proprietor: Ingenius Biotech S.L., 50018 Zaragoza (ES)
(72) Inventor: PÉREZ LÓPEZ, Miguel Ángel, 50018 Zaragoza (ES)
(74) Representative: Schäfer, Matthias W.
(86) International application number: PCT/ES2017/070629
(87) International publication number: WO 2018/069557

(56) References cited:
- EP-A1- 2 420 147
- ES-T3- 2 201 467
- US-A1- 2002 182 196
- US-A1- 2006 257 502
- US-A1- 2009 017 136
- US-A1- 2009 104 171
- US-A1- 2015 296 853
- US-A1- 2016 030 311

## Description

### Object of the invention

Dietary supplement composition, comprising:
- (A) at least one agent that promotes the synthesis of ATP and/or creatinine phosphate in the body, wherein the component (A) comprises one or more members selected from the group consisting of nicotinamide adenine dinucleotide (NADH) and NAD+,
- (B) at least one antioxidant for reducing or eliminating the free radicals in at least one pathway in the body and normalizing the balance of oxidative stress, thereby improving cellular mitochondrial function,
- (C) at least one agent for normalizing or maintaining normal neurotransmitter function, normal protein synthesis, psychological function and normal muscle contraction in the body, wherein the component (C) comprises one or more members selected from the group consisting of magnesium citrate and magnesium malate,
- (D) at least one agent for normalizing or maintaining the production of serotonin, melatonin and niacin in the human body, wherein the component (D) comprises one or more members selected from the group consisting of tryptophan, L-tryptophan and L-5-hydroxytryptophan, 5-HTP, as claimed.

And also, typical excipients and components necessary for its formulation as a dietary supplement.

The dietary supplement component, object of the present invention, has applications such as to normalize alteration or deterioration of neurological function and the normal function of the mitochondrial structure in the human body, improving general energy performance, concentration and physical stress resistance, thereby contributing to the improvement of mood in situations of stress, in addition to contributing to minimizing the side effects in anticholesterol treatments with statins.

### Background of the invention

The existence is known of nutritional supplements, e.g. to reduce physical and mental fatigue, to enhance and improve activity, promote muscle performance, increase energy substrates, contribute to improving antioxidant defences, e.g. patent DE10326822 discloses a dietary supplement that comprises NADH, L-camitine, co-enzyme Q10, L-carnosine, succinic acid, ascorbic acid, bioflavonoids and/or their derivatives, wherein it also includes a pharmaceutical preparation which comprises the dietary supplement and excipients. Of activity: anti-atherosclerotic and anti-asthmatic.

Another nutritional supplement composition is disclosed in patent P201001081 which comprises NADH and/or NAD+, co-enzyme Q10, vitamin C (ascorbic acid), serine and/or phosphoserine, and typical excipients necessary for its formulation, particularly advantageous for professional drivers and people who normally take drugs that have somnolence as main side effect.

Patent ES2038603 discloses the use of oligopeptides for the treatment of brain disorders, especially insomnia and depressions, in which formula it includes, among others, a residue derived from tryptophan, from 5-hydroxy-L-tryptophan or of one of the L-amino acids: glycine, alanine, serine, threonine, cysteine, methionine, asparagine, aspartic acid, glutamine, glutamic acid, histidine, lysine and proline.

Patent ES2087027 relates to nutritional supplements that confer thermogenic character to the food or diets that contain them when they are ingested by people, causing most of the energy from these foods to be lost as heat (thermogenesis), being essentially constituted by one or more thermogenic amino acids selected from L-tryptophan, L-methionine, L-threonine, or combinations with the remaining essential amino acids or their precursors or derivatives selected from among their esters, neutral salts, protein hydrolysed synthetic polymers or complexes of said amino acids or mixtures rich in said amino acids.

Patent ES2190069 relates to solid compositions appropriate for administration by oral route which contain a magnesium citrate of alkanoyl L-carnitine, to provide stable and non-hygroscopic salts of lower alkanoyl L-carnitine which have an enhanced nutritional and/or therapeutic efficacy with respect to their counterparts in the internal salts.

Patent ES2284900 discloses a dietary or health supplement comprising an effective quantity of a micronutrient selected from the group consisting of complexes of derivatives of ubiquinol phosphate, ascorbic acid, retinol, tocotrienol, tocopherol and mixtures thereof administered with an acceptable vehicle, wherein the micronutrient is reaction product of (a) one or more derivatives of ubiquinol phosphate, ascorbic acid, tocotrienol, retinol, tocopherol and mixtures thereof and (b) one or more complexing agents selected from the group consisting of amphoteric surfactants, cationic surfactants, amino acids which have functional groups of nitrogen and proteins rich in these amino acids.

Patent ES2311297 relates to an amino acid supplement for patients in dialysis for the treatment of hypoalbuminemia, comprising in the formulation of each tablet L-histidine, L-isoleucine, L-leucine, L-lysine, L-methionine, L-phenylalanine, L-threonine, L-tryptophan, L-tyrosine and L-valine.

Patent US2002182196 relates to a composition for normalization of the alteration or deterioration of neurological function in the human body, comprising effective amounts of: (A) at least one agent promoting the synthesis of ATP and/or creatine phosphate in the body; (B) at least one antioxidant to scavenge free radicals in at least one pathway in the body; (C) at least one agent for normalizing or maintaining membrane function and structure in the body; (D) at least one agent for normalizing or maintaining normal neurotransmitter function in the body; (E) at least one agent for cortisol action down regulation; and (F) at least one agent for suppressing activation of apoptotic pathways in the human body. Similar prior art is disclosed in US 2006/257502 A1, US 2009/017136 A1 and US 2009/104171 A1.

### Dietary supplement

It relates to a dietary supplement composition with applications such as stimulating/improving/increasing the production of serotonin and adenosine triphosphate (ATP), which is produced naturally, in this way increasing general energy performance, concentration and physical stress resistance, improving the mood in situations of stress to
- reduce physical and mental fatigue,
- improve activity,
- improve recovery from activity,
- promote muscle performance,
- increase energy substrates and
- contribute to improvement of antioxidant defences (i.e. reducing oxidative stress or lipid peroxidation, preserve antioxidants in the serum), to improve the mood, comprising:
   (A) at least one agent that promotes the synthesis of ATP and/or creatinine phosphate in the body, wherein the component (A) comprises one or more members selected from the group consisting of nicotinamide adenine dinucleotide (NADH) and NAD+,
   (B) at least one antioxidant for reducing or eliminating the free radicals in at least one pathway in the body and normalizing the balance of oxidative stress, thereby improving cellular mitochondrial function,
   (C) at least one agent for normalizing or maintaining normal neurotransmitter function, normal protein synthesis, psychological function and normal muscle contraction in the body, wherein the component (C) comprises one or more members selected from the group consisting of magnesium citrate and magnesium malate,
   (D) at least one agent for normalizing or maintaining the production of serotonin, melatonin and niacin in the human body, wherein the component (D) comprises one or more members selected from the group consisting of tryptophan, L-tryptophan and L-5-hydroxytryptophan, 5-HTP, as claimed.

### (A) - comprises one or more members selected from the group consisting of nicotinamide adenine dinucleotide (NADH) and/or NAD₊.

The NADH and/or NDA+ molecule: called nicotinamide adenine dinucleotide, NAD+ abbreviated, is a co-enzyme found in all live cells. The compound is a dinucleotide, since it consists of two nucleotides bound through their phosphate groups, with a nucleotide containing an adenine base and the other containing nicotinamide.

In the human metabolism, NAD+ or NADH is involved in redox reactions, carrying electrons from one reaction to another. The co-enzyme is found in two forms in the cells: NAD+, which is an oxidant agent which accepts electrons from other molecules and is reduced to the formation of NADH, which then can be used as a reducing agent to donate electrons.

These electron transfer reactions are the main function of NAD+/NADH. However, it is also used in other cell processes, in particular as an enzyme substrate which adds or eliminates chemical groups from proteins. Due to the importance of these functions, the enzymes involved in NAD+/NADH metabolism are objectives for the discovery of drugs.

In our organism, NAD+/NADH can be synthesized from simple building blocks (de novo) from the amino acid tryptophan or aspartic acid.

Alternatively, the most complex components of the co-enzymes are taken from food such as the vitamin called niacin or vitamin B3.

Other alternative pathways with great antioxidant power are: lipoic acid, phosphocreatinine, alpha ribose, which also intervenes in the synthesis of cellular energy (ATP).

NADH is directly involved in the body's cellular defence immune system.

NADH, biologically known as co-enzyme 1 (since it is the most important co-enzyme, also known as Co-E1), is, therefore, necessary for thousands of biochemical reactions in the body and is naturally found in all live cells, as cited in the summary of the academic article:
- *Review focusing on characterization, efficacy, safety, use and promising areas for oral NADH***: Claudia Kelley, MPH, MS, HD, RD, CDE, CNSD****Professor Clinical Nutrition Science; Southern California University of Health Sciences, Whittier CA.*

As a dietary supplement, NADH is currently marketed to help increase energy and vitality in the human body. Nutritional compositions containing NADH may also help in the fight against the effects of disorders such as fibromyalgia, which is a physical disorder that makes the sensory system more sensitive to physical and environmental stimuli such as pain and cures with a neurotransmitter deficit, as cited in the summary of the following academic article:
- *Psicobioquimica (estrés, ansiedad and depresión) in fibromyalgia (Psychobiochemistry (stress, anxiety and depression): Pedro T. Sánchez, Julia M. Sánchez, Miguel de Lamo, Gema Peiró.*

To relieve the symptoms of certain energy disorders such as fatigue, chronic fatigue syndrome in particular, which is often related to stress, and is defined as a condition wherein a patient has persistent fatigue not attributable to any other cause as cited in the summaries of the following academic articles:
- *Effect of coenzyme Q10 plus nicotinamide adenine dinucleotide supplementation on maximum heart rate after exercise testing in chronic fatigue syndrome e A randomized, controlled, double-blind trial: Jesus Castro-Marrero a,* **, 1, Naia Saez-Franca s b, d, 1, Maria Jose Segundo c, Natalia Calvo, Monica Faro a, Luisa Aliste a, Tomas Fdez de Sevilla a, Jose Alegre.*
- *Does Oral Coenzyme Q 10 Plus NADH Supplementation Improve Fatigue and Biochemical Parameters in Chronic Fatigue Syndrome: Jesús Castro-Marrero, Mario D. Cordero, Maria J. Segundo, Naia Sáez-francás, Natalia Calvo, Lourdes Román-Malo, Luisa Aliste, Paula Riera Rios, Tomás Fdez. de Sevilla, José Alegre-Martin.*

### (B) one or more members selected from the group consisting of co-enzyme Q10, ubiquinone, ubiquinol; riboflavin; selenium; vitamin C; vitamin E; zinc.

Co-enzyme Q10 is also known as ubiquinone, ubidecarenone, co-enzyme Q, and abbreviated on occasions as CoQ, CoQ10, Q10, or Q. This compound is a compound of 1,4-benzoquinone, where Q refers to the chemical group of quinone and 10 refers to the number of isoprenyl chemical subunits.

This liposoluble substance is present in most eukaryote cells, mainly in the mitochondria. It is a component of the electron transport chain and participates in aerobic cell respiration, the generation of energy in the form of ATP.

Due to its capacity to transfer electrons and, therefore, act as an antioxidant, CoQ10 is used as a dietary supplement, e.g. in the following applications: mitochondrial disorders, cardiac insufficiency, headaches, cardiac arrest, blood pressure, periodontal disease, depression, Parkinson's disease, as cited in the summaries of the following academic articles:
- *Clinical applications of coenzyme Q10: Juan Garrido-Maraver, Mario D Cordero, Manuel Oropesa Avila, Alejandro Fernandez Vega, Mario De La Mata, Ana Delgado Pavon, Elisabet Alcocer-Gomez, Carmen Perez Calero, Marina Villanueva Paz, Macarena Alanis, Isabel De Lavera, David Cotan, Jose A Sanchez-Alcazar.*
- *Does Oral Coenzyme Q 10 Plus NADH Supplementation Improve Fatigue and Biochemical Parameters in Chronic Fatigue Syndrome: Jesús Castro-Marrero, Mario D. Cordero, Maria J. Segundo, Naia Sáez-francás, Natalia Calvo, Lourdes Román-Malo, Luisa Aliste, Paula Riera Rios, Tomás Fdez. de Sevilla, José Alegre-Martin.*
- *Lower plasma Coenzyme Q10 in depression: a marker for treatment resistance and chronic fatigue in depression and a risk factor to cardiovascular disorder in that illness: Michael Maes, Ivanka Mihaylova, Marta Kubera, Marc Uytterhoeven, Nicolas Vrydags, Eugene Bosmans.*

Ubiquinone, or co-enzyme Q10, is an essential component of mitochondria, performing a leading role in the electron transport system as antioxidant agent as it participates in the aerobic respiration cell mechanism and provides around 95% of the body's energy. Its deficit would be associated with a deficiency in oxidative phosphorylation and mitochondrial ATP production, harming the muscle energy metabolism and thus contributing to the development of myopathy and muscle symptoms.

Statins reduce the reserves of ubiquinone and mevalonate, reflected in reduced concentrations thereof, both on a plasma level and reserve level by 25 to 50%. The co-enzyme Q10 supplement has had beneficial effects on the systems related to myopathy mediated by the consumption of statins as cited in the summary of the following academic article:
- *Effect of Coenzyme Q10 on Myopathic Symptoms in Patients Treated With Statins*: *Giuseppe Caso, MD, MSc, PhDa, Patricia Kelly, DOb, Margaret A. McNurlan, PhDa, and William E. Lawson, MD.*

The compositions containing both NADH and CoQ10 are also known as a dietary supplement, as indicated due to their capacity to supply energy, imitating energy production mechanisms and the antioxidant activity generally produced in the human body.

Other alternative pathways for energy formation is riboflavin or vitamin B2 the same as vitamins C and E, which together with certain minerals such as zinc and selenium are vital co-factors for anti-Redox processes and ATP formation.

### (C) comprises one or more antioxidants selected from the following: magnesium citrate, magnesium malate.

Magnesium is a mineral nutrient, acting as essential co-factor in more than 300 biosynthesis processes in our body, it forms part of the Mg-ATP complex, it is essential for oxidative phosphorylation and has an important role in the energy metabolism, mineral homeostasis, calcium metabolism and neuromuscular and endocrine function.

It is also related to protein synthesis, cell mitosis, normal functioning of the nervous system and muscle contractility. In short, the relation has been established between magnesium dietary intake and the balance of electrolytes, it contributes to normal energy-yielding metabolism, normal neurotransmission and muscle contractions, including the heart muscle, normal cell division, the maintenance of normal bone, the maintenance of normal teeth normal protein synthesis, as cited in the summaries of the following academic articles:
- *EFSA Panel on Dietetic Products, Nutrition and Allergies (NDA)* (*European Food Safety Authority (EFSA), Parma, Italy, EFSA Journal 2009; 7(9):1216 Scientific Opinion on the substantiation of health claims related to magnesium and electrolyte balance (ID 238), energy-yielding metabolism (ID 240, 247, 248), neurotransmitter and muscle contraction including heart muscle (ID 241, 242), cell division (ID 365), maintenance of bone (ID 239), maintenance of teeth (ID 239), blood coagulation (ID 357) and protein synthesis (ID 364) pursuant to Article 13(1) of Regulation (EC) No 1924*/*20061.*
- *Is magnesium citrate treatment effective on pain, clinical parameters and functional status in patients with fibromyalgia?: Selda Bagis, Mehmet Karabiber, Ismet As* • *Lulu fer Tamer, Canan Erdogan, Ayçe Atalay.*

There are other molecules whose action is related or similar in its function with metabolic processes where magnesium intervenes such as, for example, flavonoids, arginine, cysteine etc. However, they have a more particular and defined role, and do not have the broad activity and metabolic necessity of magnesium.

### (D) comprises one or more members selected from of the group consisting of tryptophan, L-tryptophan, L-5 hydroxytryptophan; 5-HTP.

Tryptophan is abbreviated Trp or W in the three or one letter classification, respectively. It is one of the 20 essential amino acids for the human body, since the human species is incapable of synthesizing it ourselves, so it must be present in our diet.

Trp is an apolar, hydrophobic and aromatic amino acid, since it has a ring with conjugated double bonds, which gives the molecule great structural stability and gives off less energy in reactions in which it participates.

This aromatic quality is due to its R group (the specific side chain of the amino acid). Since it is an indole group, with two rings one of six carbons and another of five carbons, both with delocalized bonds.

Its molecular formula is C¹¹H¹²N²O², which gives it a molecular mass of 204.23 u.

It is essential for the formation of proteins and its secondary mechanism also participates in the synthesis of alkaloids, hormones and pigments. The synthesis of serotonin and its neuronal concentration directly depend on the tryptophan concentration.

Trp is a precursor of these molecules so that if we do not obtain sufficient in our diet, we cannot produce serotonin. Trp is mainly metabolized via two pathways, that of serotonin and the kynurenine pathway.

In the serotonin pathway, it is an indispensable factor in the secretion of serotonin in the pineal gland. Serotonin is a neurotransmitter that regulates highly varied processes such as sleep, stress or hunger, it is the neurotransmitter of positivism, as cited in the summaries of the following academic articles:
- *Kynurenine pathway pathologies: do nicotinamide and Other pathway co-Factors have Therapeutic Role in Reduction of symptom severity, Including chronic Fatigue syndrome (cFs) and Fibromyalgia (FM)*): *Adele Blank eld.*
- *Tryptophan Biochemistry: Structural, Nutritional, Metabolic, and Medical Aspects in Humans Lionella Palego,1 Laura Betti,2,3 Alessandra Rossi, 1 and Gino Giannaccini2,3.*

The kynurenine pathway has the final result of niacin, also called vitamin B3, which we can obtain from cereals or by the catabolism of Trp. Niacin, in turn, is a precursor of the NADPH and NADH molecules, which are the reducing powerhouse of the cell, which shall be used to synthesize molecules that require reducing or energy.

Other active metabolites of tryptophan are L-5-hydroxytryptophan and 5-htp, whose metabolic role is similar, although its molecular form is different.

### SUMMARY OF THE INVENTION

The present invention provides a composition which comprises different compatible active ingredients, capable of
- contributing to restoring and/or increasing general energy performance, concentration and physical stress resistance. Improving the mood in situations of stress.
- being used as dietary supplements for humans, particularly in those situations where a high level of attention and concentration are necessary.
- restoring/stimulating main biochemical pathways which are signalled to energy production and/or for increasing attention in the human body, without adverse side effects arising.
- stimulating/improving/increasing the production of serotonin of natural origin, thus resolving the problem due to the impossibility of oral serotonin administration, since through this pathway it does not pass the blood-brain barrier and its oral administration is totally ineffective.
- being used as a dietary supplement and nutritional products also in addition to the typical drugs and medicines, without having unexpected side effects.

These and also other objects are achieved by the composition of the dietary supplement having the features of claim 1. The following are active ingredients:
(A) at least one agent that promotes the synthesis of ATP and/or creatinine phosphate in the body, in a proportion between 1.38 and 2.76% of the overall weight of the composition, comprising one or more members selected from the group consisting of nicotinamide adenine dinucleotide (NADH) and/or NAD₊. With the preferred selected member being: nicotinamide adenine dinucleotide (NADH) and/or NAD⁺.
(B) at least one antioxidant for reducing or eliminating the free radicals in at least one pathway in the body and normalizing the balance of oxidative stress, thereby improving cellular mitochondrial function, in a proportion between 13.8 and 27.6% of the overall weight of the composition, comprising one or more members selected from the group consisting of co-enzyme Q10, ubiquinone, ubiquinol; riboflavin; selenium; vitamin C; vitamin E; zinc.
   With the preferred selected member being: co-enzyme Q10.
(C) at least one agent for normalizing or maintaining normal neurotransmitter function; normal protein synthesis; psychological function and normal muscle contraction in the body, in a proportion between 5.70 and 11.39% of the overall weight of the composition, comprising one or more antioxidants selected from the following: magnesium citrate, magnesium malate. With the selected member being: magnesium citrate.
(D) at least one agent for normalizing or maintaining the production of serotonin, melatonin and niacin in the human body, in a proportion between 20.69 and 41.38% of the overall weight of the composition, comprising one or more members selected from of the group consisting of tryptophan, L-tryptophan; L-5 hydroxytryptophan, 5-HTP.
   With the preferred selected member being: L-tryptophan.

The composition according to the present invention further comprises typical excipients and components necessary for its formulation as a dietary supplement.

A dietary supplement, also known as food supplement or nutritional supplement, is a preparation designed to supplement the diet and provide nutrients such a vitamins, minerals, fibre, fatty acids or amino acids, which may be lacking or not consumed in enough quantity in a person's diet. Some countries define dietary supplements as food, where are in others they are defined as drugs or natural products for health.

According to the present invention, each one of the active ingredients of the composition is essential for showing a surprising effect as a dietary supplement (nutritional) due to the synergic effect of its essential active ingredients and, in particular, due to the addition of L-tryptophan to the other components, i.e. NADH and/or NAD+, co-enzyme Q10 and magnesium.

It has indeed been observed that the composition according to the present invention may positively influence the main processes of natural origin of oxidation-reduction, the transfer of electrons to the enzymes that act as biocatalysts in these reactions.

For example, one of these reactions is the hydroxylation of dopamine to give noradrenaline and its process to give adrenaline as final product.

Adrenaline has a significant sympathomimetic effect, for example during emotional crisis, or when it is necessary to alert or prepare the body for an emergency situation. Indeed, adrenaline acts as a sympathetic system stimulant and is capable of producing positive effects on the central nervous system.

It must be indicated that adrenaline cannot be administered by oral route, since it undergoes oxidation and degradation/conjugation during the gastrointestinal passage and in the liver.

Therefore, it has been found, according to the present invention, that a dietary supplement composition comprising L-tryptophan, NADH or NAD+, co-enzyme Q10 and magnesium citrate) as active and the essential components surprisingly can and significantly promote the natural production of adrenaline and serotonin in the human body, whilst avoiding undesired side effects.

According to the present invention, therefore, the composition comprising L-tryptophan, NADH and/or NAD+, co-enzyme Q10 and magnesium citrate as active and essential ingredients are advantageously used as a dietary supplement in cases where it is necessary to improve and/or recover energy, for example during a convalescence, fibromyalgia, chronic fatigue syndrome, as cited in the summary of the following academic article:
- *Oxidative stress, mitochondrial dysfunction and, inflammation common events in skin of patients with Fibromyalgia: Benito Sánchez -Dguez, Pedro Bullón, Lourdes Román-Malo, Fabiola Marín-Aguilar, Elisabet Alcocer-Gómez, Angel M. Carrión, José Antonio Sánchez-Alcazar, Mario D. Cordero.*

In this case, for example, it has been observed that the composition according to the invention acts as a stimulator in cases of weakness, fatigue, tiredness and similar.

For example, always according to the invention, the composition can be advantageously used as an adjuvant dietary supplement for those people who need special attention, such as patients under treatment with statins, fibromyalgia, chronic fatigue, in performing activities and work that requires a lot of concentration and speed of response and reaction.

Another related use of the composition according to the invention is aimed, for example, at reducing the somnolence particularly caused by the intake of drugs, thus resolving a very widespread problem.

For example, those who are obliged to take antihistamines suffer very considerable problems with somnolence.

In the market, there are already associations between antihistamine and stimulant drugs; however, the exciting action of the stimulant drug necessary to contrast the side effect of an antihistaminic drug is hard to modulate and very often causes a worse side effect, keeping the patient awake too long and thus causing an associated fatigue syndrome.

The association of the intake of antihistaminic drugs with a dietary supplement, according to the present invention, would allow physiologically modulating the stimulating effect, thus avoiding an excessive response and the presence of additional side effects.

According to the present invention, the expression "association" or "combination" means that the composition according to the invention and the antihistamine (or other existing drug that has somnolence as main side effect) are administered together in separate forms as different formulations, or at different times also in separate forms and different formulations or in a single form or formulation.

The availability of a product capable of inducing a natural stimulating and endogenous effect, which may effectively contrast the side effect of somnolence of certain drugs, has been a need for some time, especially for those who must drive cars, lorries, trains and planes for professional reasons and work which requires concentration and speed of response and reaction.

The composition according to the present invention represents a large improvement in the dietary supplements designed to act in restoring/inducing energy in the human body. Indeed, the surprising fact of reducing somnolence at the same time as it induces a feeling of energy, would result in an innovative nutritional supplement with respect to the existing products, which are active in energy levels but have no effect with respect to somnolence.

It has to be indicated, as previously mentioned, that the composition according to the invention, is capable of inducing a positive response of the human body with respect to somnolence, without causing adverse side effects of fatigue or similar, as the body's response is of a "natural", "endogenous" type and is not caused by additional drugs.

Consequently, the suggested use of this dietary supplement is in all cases requiring an increase/restoration of the body's energy, together with a recovery of the attention levels affected by various causes, among which we have, for example, the intake of drugs with adverse side effects related to somnolence.

The person skilled in the art shall easily understand that he/she can combine characteristics of different embodiments with characteristics of other possible embodiments, provided that this combination is technically possible.

Always according to the invention, some examples of compositions that contain L-tryptophan, NADH and/or NAD+, co-enzyme Q10 and magnesium citrate as active ingredients, as indicated by Table 1.

***Table 1**

| **Active ingredient** | **Formula 1** | **Formula 2** | **Formula 3** |
|---|---|---|---|
| | **mg** | **mg** | **mg** |
| **L-tryptophan** | 300 | 150 | 75 |
| **CoQ10** | 200 | 100 | 50 |
| **NADH** | 20 | 10 | 5-7 |
| **magnesium citrate** | 590 | 295 | 147-150 |
| **Excipients q.s.p Approx.** | 100 | 50 | 25 |

It is intended that the previous formulations are only given as examples of dietary supplement compositions according to the invention and which do not represent in any way a limitation of the scope of protection.

The dietary supplement compositions according to the invention can be formulated in all the suitable forms for oral administration, e.g. tablets, film-coated tablets, sublingual tablets, dispersible tablets, buccal dispersible tablets, sachets, capsules, powders, granules, micro-granules, soluble effervescent granules.

Always according to the invention, the dietary supplement compositions may also be formulated in all the suitable forms for rectal administration, nasal administration or any other suitable administration.

The information contained in examples, modes of embodiment and tables forms part of the description of the invention.

## Claims

1. Dietary supplement composition, **characterized in that** it comprises:
• (A) at least one agent that promotes the synthesis of ATP and/or creatinine phosphate in the body, in a proportion between 1.38 and 2.76% of the overall weight of the composition, wherein the component (**A**) comprises one or more members selected from the group consisting of nicotinamide adenine dinucleotide (NADH) and NAD⁺,
• (B) at least one antioxidant for reducing or eliminating the free radicals in at least one pathway in the body and normalizing the balance of oxidative stress, thereby improving cellular mitochondrial function, in a proportion between 13.8 and 27.6% of the overall weight of the composition,
• (C) at least one agent for normalizing or maintaining normal neurotransmitter function; normal protein synthesis; psychological function and normal muscle contraction in the body, in a proportion between 5.70 and 11.39% of the overall weight of the composition, wherein the component (**C**) comprises one or more members selected from the group consisting of magnesium citrate and magnesium malate,
• (D) at least one agent for normalizing or maintaining the production of serotonin, melatonin and niacin in the human body, in a proportion between 20.69 and 41.38% of the overall weight of the composition, wherein the component (**D**) comprises one or more members selected from the group consisting of tryptophan, L-tryptophan and L-5-hydroxytryptophan, 5-HTP,
• wherein the component (**B**) comprises one or more members selected from the group consisting of co-enzyme Q10 or ubiquinone, ubiquinol, riboflavin, selenium, vitamin C, vitamin E, zinc..

2. Dietary supplement composition, according to the preceding claim, **characterized in that** the component (**B**) selected is co-enzyme Q10 or ubiquinone.

3. Dietary supplement composition, according to the preceding claims, **characterized in that** the component (**C**) selected is magnesium citrate.

4. Dietary supplement composition, according to the preceding claims, **characterized in that** the component (**D**) selected is L-tryptophan.

## Patentansprüche

1. Nahrungsergänzungszusammensetzung, **dadurch gekennzeichnet, dass** sie aufweist:
• (A) mindestens einen Wirkstoff, der die Synthese von ATP und/oder Kreatininphosphat im Körper fördert, in einem Verhältnis zwischen 1,38 und 2,76 % des Gesamtgewichts der Zusammensetzung, wobei die Komponente (A) ein oder mehrere Elemente aufweist, die aus der aus Nikotinamid-Adenin-Dinukleotid (NADH) und NAD+ bestehenden Gruppe ausgewählt sind,
• (B) mindestens ein Antioxidans zur Verringerung oder Beseitigung der freien Radikale in mindestens einem Weg im Körper und zur Normalisierung des Gleichgewichts von oxidativem Stress, wodurch die zelluläre mitochondriale Funktion verbessert wird, in einem Verhältnis zwischen 13,8 und 27,6 % des Gesamtgewichts der Zusammensetzung,
• (C) mindestens einen Wirkstoff zur Normalisierung oder Aufrechterhaltung normaler Neurotransmitterfunktion, normaler Proteinsynthese, psychologischer Funktion und normaler Muskelkontraktion im Körper in einem Verhältnis zwischen 5,70 und 11,39 % des Gesamtgewichts der Zusammensetzung, wobei die Komponente (C) ein oder mehrere Elemente aufweist, die aus der aus Magnesiumcitrat und Magnesiummalat bestehenden Gruppe ausgewählt sind,
• (D) mindestens einen Wirkstoff zur Normalisierung oder Aufrechterhaltung der Produktion von Serotonin, Melatonin und Niacin im menschlichen Körper, in einem Verhältnis zwischen 20,69 und 41,38 % des Gesamtgewichts der Zusammensetzung, wobei die Komponente (D) ein oder mehrere Elemente aufweist, die aus der aus Tryptophan, L-Tryptophan und L-5-Hydroxytryptophan, 5-HTP bestehenden Gruppe ausgewählt sind,
• wobei die Komponente (B) ein oder mehrere Elemente aufweist, die aus der aus CoEnzym Q10 oder Ubichinon, Ubiquinol, Riboflavin, Selen, Vitamin C, Vitamin E, Zink bestehende Gruppe ausgewählt sind.

2. Nahrungsergänzungsmittelzusammensetzung nach dem vorhergehenden Anspruch, **dadurch gekennzeichnet, dass** die ausgewählte Komponente (B) Coenzym Q10 oder Ubichinon ist.

3. Nahrungsergänzungsmittelzusammensetzung nach den vorhergehenden Ansprüchen, **dadurch gekennzeichnet, dass** die ausgewählte Komponente (C) Magnesiumcitrat ist.

4. Nahrungsergänzungsmittelzusammensetzung nach dem vorhergehenden Anspruch, **dadurch gekennzeichnet, dass** die ausgewählte Komponente (D) L-Tryptophan ist.

## Revendications

1. Composition de complément diététique, **caractérisée en ce qu'**elle comprend :
• (A) au moins un agent qui promeut la synthèse de l'ATP et/ou la phospho-créatinine dans le corps, dans une proportion comprise entre 1,38 et 2,76 % du poids total de la composition, dans laquelle le constituant (A) comprend un ou plusieurs éléments choisis dans le groupe composé de nicotinamide adénine dinucléotide (NADH) et/ou NAD⁺,
• (B) au moins un antioxydant destiné à réduire ou éliminer les radicaux libres dans au moins un chemin dans le corps et normaliser l'équilibre du stress oxydant, en améliorant ainsi la fonction mitochondriale cellulaire, dans une proportion comprise entre 13,8 et 27,6 % du poids total de la composition,
• (C) au moins un agent destiné à normaliser ou maintenir la fonction normale des neurotransmetteurs ; la synthèse normale des protéines ; la fonction psychologique et la contraction normale des muscles dans le corps, dans une proportion comprise entre 5,70 et 11,39 % du poids total de la composition, dans laquelle le constituant (C) comprend un ou plusieurs éléments choisis dans le groupe composé de citrate de magnésium et de malate de magnésium,
• (D) au moins un agent destiné à normaliser ou maintenir la production de sérotonine, de mélatonine et de niacine dans le corps humain, dans une proportion comprise entre 20,69 et 41,38 % du poids total de la composition, dans laquelle le constituant (D) comprend un ou plusieurs éléments choisis dans le groupe composé de tryptophane, L-tryptophane et L-5 hydroxytryptophane, 5-HTP,
• dans laquelle le constituant (B) comprend un ou plusieurs éléments choisis dans le groupe composé de co-enzyme Q10 ou ubiquinone, ubiquinol, riboflavine, sélénium, vitamine C, vitamine E, zinc.

2. Composition de complément diététique selon la revendication précédente, **caractérisée en ce que** le constituant (B) choisi est la co-enzyme Q10 ou l'ubiquinone.

3. Composition de complément diététique selon les revendications précédentes, **caractérisée en ce que** le constituant (C) choisi est le citrate de magnésium.

4. Composition de complément diététique selon les revendications précédentes, **caractérisée en ce que** le constituant (D) choisi est le L-tryptophane.
